(19) Europäisches Patentamt
European Patent Office
Office européen des brevets

(11) EP 0 635 500 B1

# (12) EUROPÄISCHE PATENTSCHRIFT

(45) Veröffentlichungstag und Bekanntmachung des Hinweises auf die Patenterteilung:
**22.03.2000 Patentblatt 2000/12**

(21) Anmeldenummer: **94110671.8**

(22) Anmeldetag: **08.07.1994**

(51) Int. Cl.[7]: **C07D 265/08**, C07D 265/12, C07D 279/06, C07D 498/10, C07D 413/10, C07D 413/06, A01N 43/86
// (C07D498/10, 319:00, 265:00)

## (54) 5,6-Dihydro-(4H)1,3-Oxazin und - Thiazin-Derivate als Insektizide

5,6-Dihydro-(4H)1,3-oxazine and -thiazine derivatives as insecticides

Dérivés de 5,6-dihydro-(4H)1,3-oxazine et -thiazine comme insecticides

(84) Benannte Vertragsstaaten:
**BE CH DE DK ES FR GB GR IT LI NL PT**

(30) Priorität: **21.07.1993 JP 20016893**
**02.12.1993 DE 4341065**

(43) Veröffentlichungstag der Anmeldung:
**25.01.1995 Patentblatt 1995/04**

(73) Patentinhaber:
- **YASHIMA CHEMICAL INDUSTRY CO., LTD.**
  **Kawasaki-shi Kanagawa 213-0002 (JP)**
- **BAYER AG**
  **51368 Leverkusen (DE)**

(72) Erfinder:
- **Itoh, Yoshiaki**
  **Nagano-shi, Nagano-ken (JP)**
- **Ishida, Tatsuya**
  **Nagano-shi, Nagano-ken (JP)**
- **Kikuchi, Yasuo**
  **Nagano-shi, Nagano-ken (JP)**
- **Suzuki, Junji**
  **Suzuka-shi, Nagano-ken (JP)**
- **Morikawa, Chiharu**
  **Suzuka-shi, Nagano-ken (JP)**
- **Tsukidate, Yokichi**
  **Nagano-shi, Nagano-ken (JP)**
- **Kudoh, Kichizo**
  **Nagano-shi, Nagano-ken (JP)**
- **Holmwood, Graham, Dr.**
  **D-42327 Wuppertal (JP)**
- **Kraatz, Udo, Dr.**
  **D-51375 Leverkusen (JP)**
- **Wachendorff-Neumann, Ulrike Dr.**
  **D-40789 Monheim (JP)**
- **Erdelen, Christoph, Dr.**
  **D-42799 Leichlingen (JP)**

(74) Vertreter:
**Schumacher, Günter, Dr.**
**Bayer AG**
**Konzernbereich RP**
**Patente und Lizenzen**
**51368 Leverkusen (DE)**

(56) Entgegenhaltungen:
**EP-A- 0 226 837**
**DE-A- 2 049 160**
**FR-A- 1 582 751**
**GB-A- 2 185 978**
**US-A- 3 450 699**
**WO-A-94/14783**
**FR-A- 1 478 076**
**FR-A- 2 158 143**
**US-A- 3 397 201**

- **JOURNAL OF THE CHEMICAL SOCIETY, PERKIN TRANSACTIONS 1, Nr.8, 1973, LETCHWORTH GB Seiten 771 - 774 LUIGI ABIS ET AL. 'A new stereospecific synthesis of 5,6-dihydro 4H-1,3-thiazines by polar 1,4-cycloaddition of thioamidoalkyl ions to olefins.'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Xerox (UK) Business Services
2.16.7/3.6

## Beschreibung

Die Erfindung betrifft die Verwendung von neuen Azin-Derivaten zur Bekämpfung von tierischen Schädlingen.

Es ist bereits bekannt, daß bestimmte Azine wie beispielsweise das 3,6-Bis-(2-chlorphenyl)-1,2,4,5-tetrazin akarizide Eigenschaften aufweisen (vgl. z.B. EP-A 0005912). Desweiteren sind aus FR-A-1 478 076 und WO-A 94/14783 Azine mit bioziden Eigenschaften bekannt. Die Wirksamkeit dieser vorbekannten Verbindungen ist jedoch, insbesondere bei niedrigen Aufwandmengen und Konzentrationen nicht in allen Anwendungsgebieten völlig zufriedenstellend.

Es wurde nun gefunden, daß die Azin-Derivate der Formel (Ia-30)

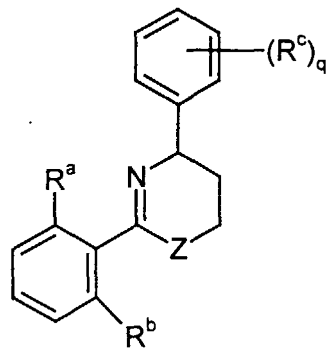

(Ia-30)

in welcher

$R^a$ für Wasserstoff, Fluor oder Chlor steht,

$R^b$ für Fluor oder Chlor steht,

q für eine Zahl von 1 bis 5 steht,

$R^c$ für Tri($C_1$-$C_6$ Alkyl)silyl, für gegebenenfalls einfach bis dreifach durch $C_1$-$C_6$ Alkyl substituiertes $C_3$-$C_7$-Cycloalkyl oder für

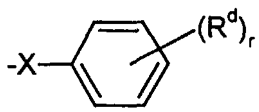

steht, worin

X für eine direkte Bindung, $C_1$-$C_6$ Alkandiyl, $C_1$-$C_6$-Oxy-Alkandiyl oder Di($C_1$-$C_6$ Alkyl)silyl steht, wobei

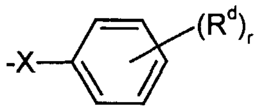

nicht für unsubstituiertes Phenylmethoxy steht,

r für eine Zahl von 0 bis 5 steht,

$R^d$ für $C_1$-$C_{12}$-Alkyl, $C_1$-$C_{12}$-Alkoxy, Halogen, $C_1$-$C_6$-Halogenalkyl, $C_1$-$C_6$-Halogenalkoxy oder Tri($C_1$-$C_6$ Alkyl)silyl steht und

Z für Sauerstoff oder Schwefel steht,
ausgenommen die Verbindung der Formel

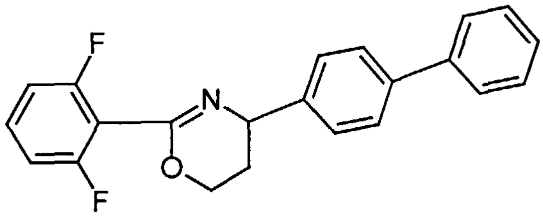

,

sehr gut zur Bekämpfung von tierischen Schädlingen, insbesondere von Arthropoden und Nematoden, geeignet sind.

Die Verbindungen der Formel (Ia-30) können in Abhängigkeit von der Art der Substituenten als geometrische und/oder optische Isomere oder Isomerengemische unterschiedlicher Zusammensetzung vorliegen. Die Erfindung betrifft sowohl die reinen Isomeren als auch die Isomerengemische.
Überraschenderweise zeigen die erfindungsgemäß verwendeten Azine der Formel (Ia-30) eine erheblich bessere Wirksamkeit gegenüber tierischen Schädlingen als die konstitutionell ähnlichsten vorbekannten Verbindungen.
Die erfindungsgemäß verwendbaren Verbindungen sind durch die Formel (Ia-30) allgemein definiert.
Weiterhin wurde gefunden, daß man die neuen Azin-Derivate der Formel (Ia-30) erhält, wenn man

a) Aminoalkohole der Formel

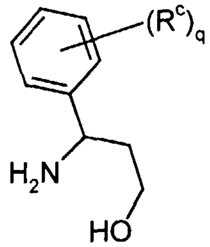

(II-30),

in welcher $R^c$ und q die in Anspruch 1 genannte Bedeutung haben, mit einer Carbonsäure der Formel

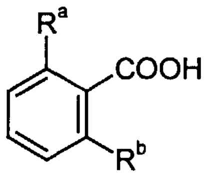

(III-30),

in welcher $R^a$ und $R^b$ die in Anspruch 1 genannten Bedeutungen haben,
mit einem wasserentziehenden Mittel, gegebenenfalls in Gegenwart eines Verdünnungsmittels umsetzt;
oder

a) Amidalkohole der Formel

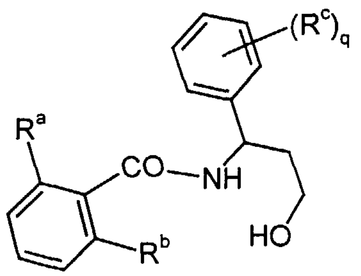

(IVa-30)

in welchen $R^a$, $R^b$, $R^c$ und q die in Anspruch 1 genannten Bedeutungen haben, mit einem wasserentziehenden Mittel, gegebenenfalls in Gegenwart eines Verdünnungsmittels umsetzt; oder

c) Amid-Derivate der Formel

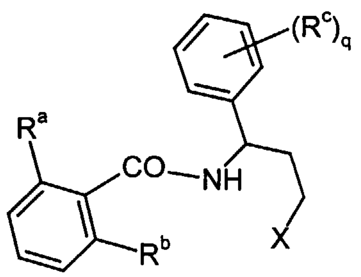

(Va-30)

in welchen $R^a$, $R^b$, $R^c$ und q die in Anspruch 1 genannten Bedeutungen haben, und X für Halogen, Alkylsulfonyloxy oder Arylsulfonyloxy steht, mit einer Base, gegebenenfalls in Gegenwart eines Verdünnungsmittels umsetzt; oder

d) Amidalkohole der Formel

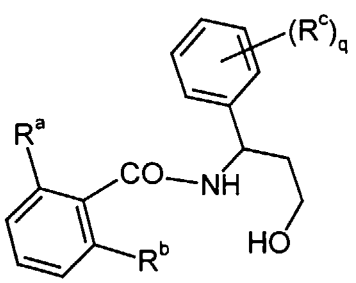

(IVa-30)

in welchen $R^a$, $R^b$, $R^c$ und q die in Anspruch 1 genannten Bedeutungen haben, mit thienylierenden Mitteln, gegebenenfalls in Gegenwart eines Verdünnungsmittels umsetzt.

Die oben bei der Definition der erfindungsgemäßen Verbindungen genannten Kohlenwasserstoffreste, wie

Alkyl, sind - auch in Verbindung mit Heteroatomen, wie Alkoxy - soweit möglich, jeweils geradkettig oder verzweigt.

In der Definition der Verbindungen der Formel (Ia-30) bedeutet der Ausdruck "Alkyl" für sich oder in Verbindung mit anderen Gruppen einen geradkettigen oder verzweigten, gesättigten aliphatischen Kohlenwasserstoffrest. Genannt seien beispielsweise Methyl, Ethyl, n-Propyl, Isopropyl, n-Butyl, Isobutyl, sec.-Butyl, tert.-Butyl, n-Pentyl, Isoamyl, Neopentyl, n-Hexyl, n-Heptyl, 1,1-Dimethylpentyl, n-Octyl, 1-Methylheptyl, 1,1-Dimethyl-4-methylpentyl, n-Nonyl, 1,1Dimethylheptyl, n-Decyl, n-Undecyl, 4,8-Dimethylnonyl und n-Dodecyl.

Als Beispiele für Halogenalkylgruppen seien genannt Chlormethyl, Difluormethyl, Bromdifluormethyl, Trifluormethyl, Fluorethyl, Trifluorethyl, Perfluorethyl.

In den Tri-($C_1$-$C_6$-Alkyl)silyl-Gruppen können die Alkylreste gleich oder verschleden sein. Genannt seien beispielsweise die Gruppen Trimethylsilyl, Ethyldimethylsilyl, n-Propyldimethylsilyl, tert.-Butyldimethylsilyl, Triethylsilyl und Methyldiethylsilyl.

Als gegebenenfalls durch $C_1$-$C_6$-Alkyl substituiertes $C_3$-$C_7$-Cycloalkyl seien beispielhaft genannt Cyclopropyl, Cyclobutyl, Cyclopentyl, Cyclohexyl, Cycloheptyl, 4-Methylcyclohexyl, 4-Ethylcyclohexyl, 4-tert.-Butylcyclohexyl.

Als Beispiele für $C_1$-$C_6$-Alkandiyl für sich oder in Verbindung mit anderen Gruppen seien genannt die Reste -$CH_2$- , -$CH_2$-$CH_2$- ,

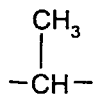 und 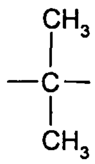 .

In der Di($C_1$-$C_6$-Alkyl)silyl können die Alkylreste gleich oder verschieden sein. Beispielhaft seien Dimethylsilyl und Diethylsilyl genannt.

Beispiele für die neuen erfindungsgemäßen Verbindungen sind in den Tabellen 1 bis 5 aufgeführt.

Tabelle 1

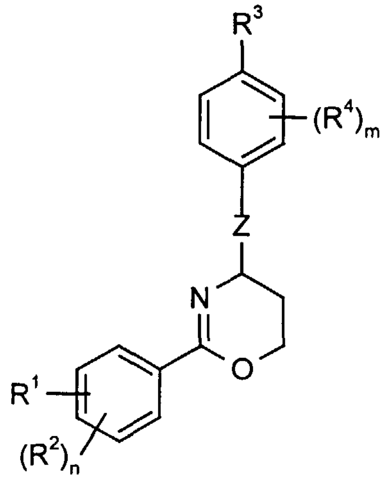

(Ia-31)

Verbindungen der Tabelle 1 entsprechen der allgemeinen Formel (Ia-31),

in welcher

$R^1$ = 2-F; $(R^2)_n$ = 6-F; $(R^4)_m$ = H; Z = direkte Bindung und $R^3$ = wie im folgenden aufgelistet.

| Verbindungs-Nr. | $R^3$ |
|---|---|
| 33 | 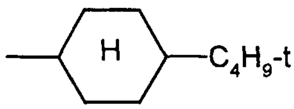 |
| 37 | 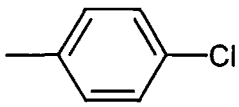 |
| 38 | 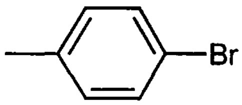 |

| Verbindungs-Nr. | $R^3$ |
|---|---|
| 39 | 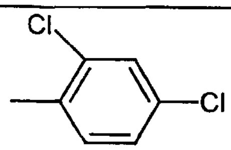 |
| 40 | 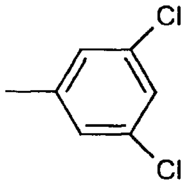 |
| 41 | 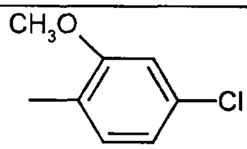 |
| 42 | 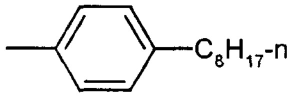 |
| 43 | 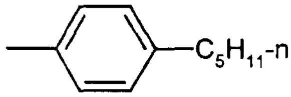 |
| 44 | 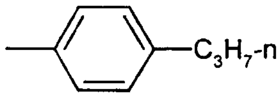 |
| 45 | 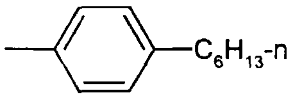 |
| 46 | 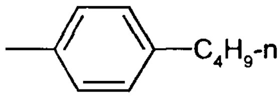 |
| 47 | 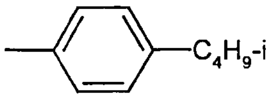 |
| 48 | 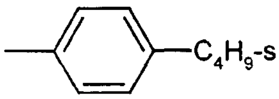 |
| 49 | 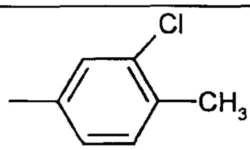 |

| Verbindungs-Nr. | $R^3$ |
|---|---|
| 50 | 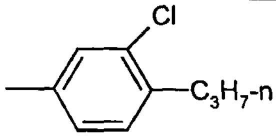 |
| 51 | 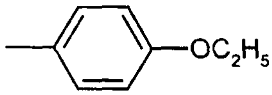 |
| 52 | 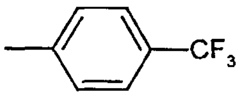 |
| 53 | 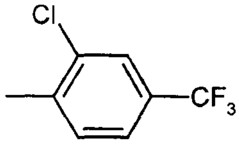 |
| 54 | 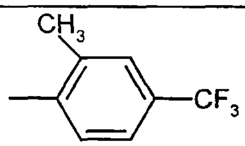 |
| 55 | 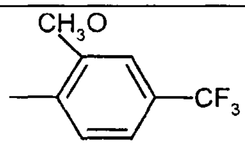 |
| 56 | 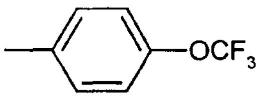 |
| 57 | 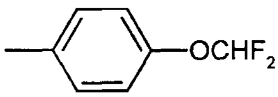 |
| 58 | 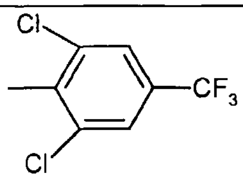 |
| 59 | 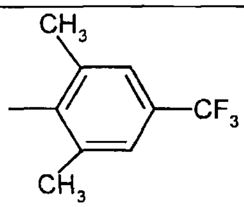 |

| Verbindungs-Nr. | $R^3$ |
|---|---|
| 60 | 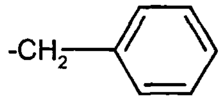 |
| 61 | 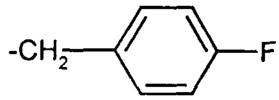 |
| 62 | 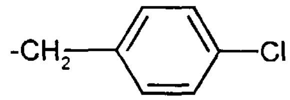 |
| 63 | 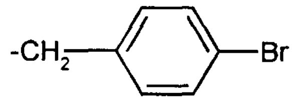 |
| 64 | 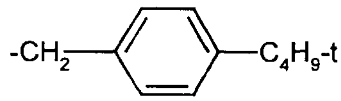 |
| 65 | 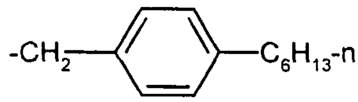 |
| 66 | 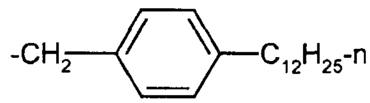 |
| 67 | 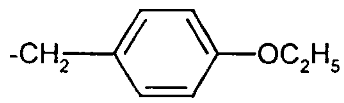 |
| 68 | 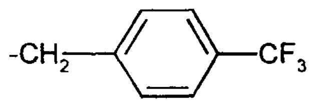 |
| 69 | 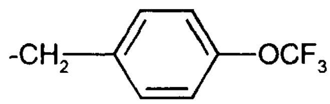 |
| 105 | 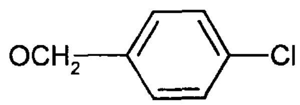 |
| 106 | 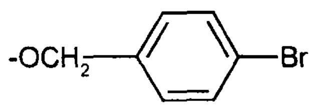 |

| Verbindungs-Nr. | $R^3$ |
|---|---|
| 107 | 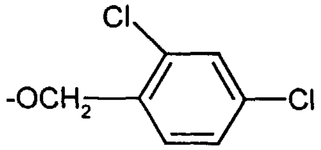 |
| 108 | 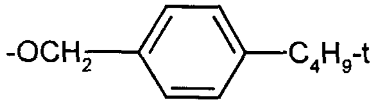 |
| 109 | 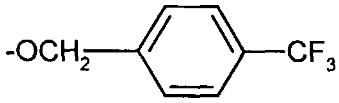 |
| 110 | 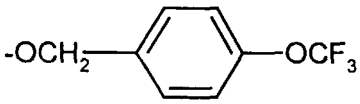 |
| 111 | 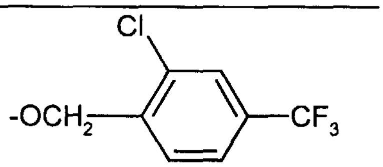 |
| 112 | 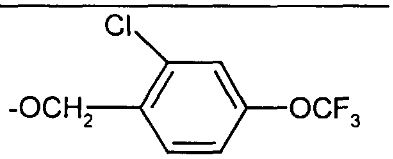 |

Tabelle 2

Die Tabelle 2 enthält die Verbindungen der allgemeinen Formel (Ia-31), in welcher $R^3$, $(R^4)_m$ und Z für die Bedeutungen der Tabelle 1 stehen und $R^1$ = 2-F; $(R^2)_n$ = 6-Cl.

Tabelle 3

Die Tabelle 3 enthält die Verbindungen der allgemeinen Formel (Ia-31), in welcher $R^3$, $(R^4)_m$ und Z für die Bedeutungen der Tabelle 1 stehen und $R^1$ = 2-Cl; $(R^2)_n$ = 6-Cl.

Tabelle 4

Die Tabelle 4 enthält die Verbindungen der allgemeinen Formel (Ia-31), in welcher $R^3$, $(R^4)_m$ und Z für die Bedeutungen der Tabelle 1 stehen und $R^1$ = 2-Cl; $(R^2)_n$ = H.

Tabelle 5

Die Tabelle 5 enthält die Verbindungen der allgemeinen Formel (Ia-31), in welcher $R^3$, $(R^4)_m$ und Z für die Bedeutungen der Tabelle 1 stehen und $R^1$ = 2-F; $(R^2)_n$ = H.

Verwendet man zur Durchführung des erfindungsgemäßen Verfahrens (a) beispielsweise 3-Amino-3-(4-fluorphenyl)-1-propanol und 2-Chlor-4-fluor-benzoesäure, so kann der Reaktionsablauf durch das folgende Formelschema skizziert werden:

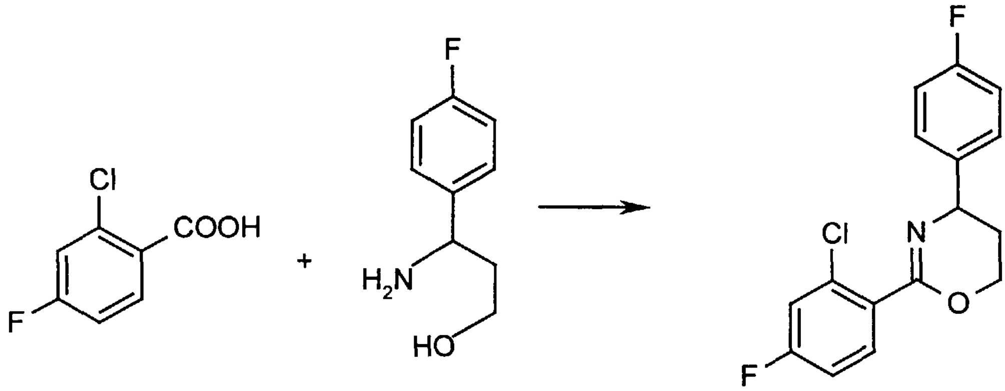

Verwendet man zur Durchführung des erfindungsgemäßen Verfahrens (b) beispielsweise N-(3-Hydroxy-1-(2-methoxy-phenyl)-propyl)-2,5-difluor-benzamid als Ausgangsverbindung und Polyphosphorsäure (PPS) als wasser-entziehendes Mittel, so kann der Reaktionsablauf durch das folgende Formelschema skizziert werden:

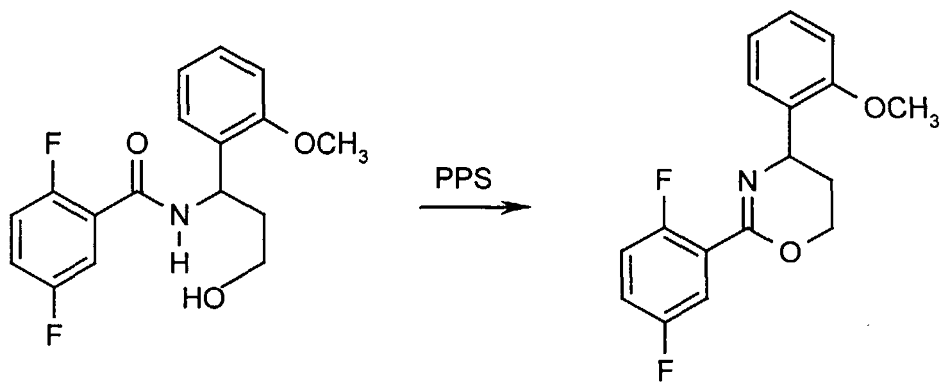


Verwendet man zur Durchführung des erfindungsgemäßen Verfahrens (c) beispielsweise N-(1-Benzyl-3-chlor-2-methyl-propyl)-2,3-difluor-benzamid als Ausgangsverbindung und Triethylamin als Base, so kann der Reaktionsablauf durch das folgende Formelschema skizziert werden:

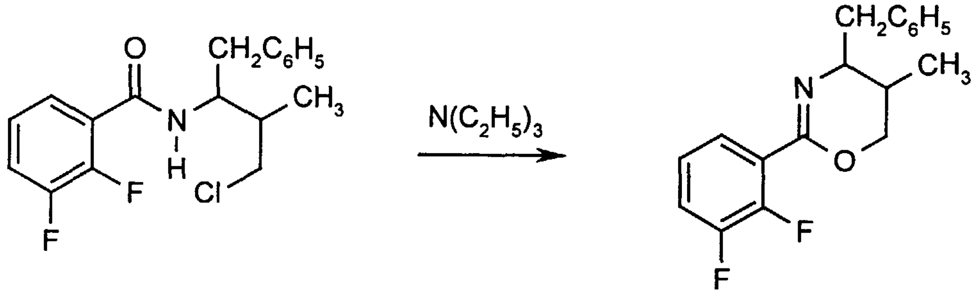


Verwendet man zur Durchführung des erfindungsgemäßen Verfahrens (d) beispielsweise N-(3-Hydroxy-1-phenoxymethyl-propyl)-2-chlor-6-fluor-benzamid als Ausgangsverbindung und Phosphor(V)-sulfid als thienylierendes Mittel, so kann der Reaktionsablauf durch das folgende Formelschema skizziert werden:

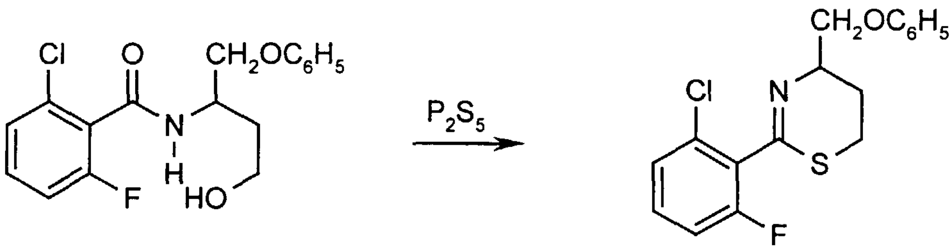


Die beim erfindungsgemäßen Verfahren (a) zur Herstellung der Verbindungen der Formel (Ia-30) als Ausgangsstoffe zu verwendenden Aminoalkohole sind durch die Formel (II-30) allgemein definiert. In der Formel (II-30) hat $(R^c)q$ die obengenannten Bedeutungen.

Die Ausgangsstoffe der Formel (II-30) sind bekannt und/oder können nach an sich bekannten Verfahren hergestellt werden (vgl. Heterocycles 9 (1978), 1277-1285; J. Org. Chem. 43 (1978), 2539-2541; Liebigs Ann. Chem. 1980, 122-139; Tetrahedron Lett. 26 (1985), 4971-4974).

Man erhält die Aminoalkohole der Formel (II-30) beispielsweise, wenn man geeignete Methoximinopropionsäureester mit Reduktionsmitteln, wie z.B. Lithiumaluminiumhydrid, gegebenenfalls in Gegenwart eines Verdünnungsmittels, wie z.B. 1,2-Dimethoxyethan, bei Temperaturen zwischen -20°C und +100°C umsetzt (vgl. J. Org. Chem. 43 (1978), 2539-2541 und die Herstellungsbeispiele).

Die hierbei als Vorprodukte benötigten Methoximinopropionsäureester können auf übliche Weise aus entsprechenden Ketoestern und O-Methyl-hydroxylamin oder dessen Hydrochlorid erhalten werden (vgl. die Herstellungsbeispiele).

Die beim erfindungsgemäßen Verfahren (a) zur Herstellung der Verbindungen der Formel (Ia-30) weiter als Ausgangsstoffe zu verwendenden Carbonsäuren sind durch die Formel (III-30) allgemein definiert. In der Formel (III-30) haben $R^a$ und $R^b$ die oben angegebenen Bedeutungen.

Die Ausgangsstoffe der Formel (III-30) sind bekannte organischen Synthesechemikalien.

Die erfindungsgemäßen Verfahren (a) und (b) werden unter Verwendung eines wasser-entziehenden Mittels durchgeführt. Es können die in der organischen Chemie üblichen wasser-entziehenden Mitttel eingesetzt werden. Vorzugsweise verwendbar sind Schwefelsäure, Polyphosphorsäure (PPS), Phosphor(V)-oxid, Dicyclohexylcarbodiimid (DCC), Phosphor(V)-sulfid und das System Triphenylphosphin/Triethylamin/Tetrachlormethan.

Als Verdünnungsmittel zur Durchführung der erfindungsgemäßen Verfahren (a) bis (d) kommen die üblichen organischen Lösungsmittel in Betracht. Vorzugsweise verwendbar sind aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlormethan; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid und N,N-Dimethylacetamid, N-Methyl-pyrrolidon oder Hexamethylphosphorsäuretriamid; Ester wie Essigsäuremethylester oder Essigsäureethylester, sowie Sulfoxide, wie Dimethylsulfoxid, gegebenenfalls auch Alkohole wie Methanol oder Ethanol.

Die Reaktionstemperaturen können bei der Durchführung des erfindungsgemäßen Verfahrens (a) in einem größeren Bereich variiert werden. Im allgemeinen arbeitet man bei Temperaturen zwischen 0°C und 150°C, vorzugsweise bei Temperaturen zwischen 10°C und 100°C.

Das erfindungsgemäße Verfahren (a) wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens (a) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines wasser-entziehenden Mittels durchgeführt, und das Reaktionsgemisch wird mehrere Stunden bei der jeweils erforderlichen Temperatur gerührt. Die Aufarbeitung erfolgt bei den erfindungsgemäßen Verfahren jeweils nach üblichen Methoden (vgl. die Herstellungsbeispiele).

In einer besonderen Ausführungsform des erfindungsgemäßen Verfahrens (a) können statt der Carbonsäuren der Formel (III-30) auch entsprechende Nitrile eingesetzt werden, wobei dann vorzugsweise an Stelle eines wasser-entziehenden Mittels ein Katalysator, wie z.B. Zink-(II)-chlorid verwendet wird.

Die bei den erfindungsgemäßen Verfahren (b) und (d) zur Herstellung der Verbindungen der Formel (Ia-30) als Ausgangsstoffe zu verwendenden Amidalkohole sind durch die Formeln (IVa-30) allgemein definiert. In der Formel (IVa-30) haben $R^a$, $R^b$ und $(R^c)q$ die obengenannten Bedeutungen.

Die Ausgangsstoffe der Formel (IVa-30) sind bekannt und/oder können nach an sich bekannten Verfahren hergestellt werden.

Man erhält die Amidalkohole der Formel (IVa-30) beispielsweise, wenn man von den Carbonsäuren der Formel (III-30) abgeleitete Säurechloride mit Aminoalkoholen der Formel (II-30) in Gegenwart eines Säurebindemittels, wie z.B. Triethylamin, Pyridin, Kaliumcarbonat, Natriumhydroxid oder Kalium-t-butylat, und in Gegenwart eines Verdünnungsmittels, wie z.B. Toluol, Chlorbenzol, Aceton oder Acetonitril, bei Temperaturen zwischen 0°C und 100°C umsetzt (vgl. die Herstellungsbeispiele).

Die Reaktionstemperaturen können bei der Durchführung des erfindungsgemäßen Verfahrens (b) in einem größeren Bereich variiert werden. Im allgemeinen arbeitet man bei Temperaturen zwischen -20°C und + 150°C, vorzugsweise bei Temperaturen zwischen 0°C und 100°C.

Das erfindungsgemäße Verfahren (b) wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens (b) zur Herstellung der Verbindungen der Formel (Ia-30) setzt man pro Mol an Amidalkohol der Formel (IVa-30) im allgemeinen 1 bis 20 Mol, vorzugsweise 1 bis 5 Mol an wasser-entziehendem Mittel ein.

In einer bevorzugten Ausführungsform des erfindungsgemäßen Verfahrens (b) wird der Amidalkohol der Formel (IVa-30) in einem Verdünnungsmittel vorgelegt und das wasser-entziehende Mittel wird dann eindosiert. Das Reaktionsgemisch wird bei der erforderlichen Temperatur bis zum Ende der Umsetzung gerührt und anschließend aufübliche Weise aufgearbeitet (vgl. die Herstellungsbeispiele).

Die beim erfindungsgemäßen Verfahren (c) zur Herstellung der Verbindungen der Formel (Ia-30) als Ausgangsstoffe zu verwendenden Amid-Derivate sind durch die Formel (Va-30) allgemein definiert. In der Formel (Va-30) haben $R^a$, $R^b$ und $(R^c)q$ die obengenannten Bedeutungen; X steht vorzugsweise für Fluor, Chlor, Brom, Iod, $C_1$-$C_4$-Alkyl-sulfonyloxy, Phenylsulfonyloxy oder Tolylsulfonyloxy, insbesondere für Chlor, Brom, Methylsulfonyloxy oder Tolylsulfonyloxy.

Die Ausgangsstoffe der Formel (Va-30) sind bekannt und/oder können nach an sich bekannten Verfahren hergestellt werden.

Man erhält die Amid-Derivate der Formel (Va-30), wenn man entsprechende Amidalkohole der Formel (IVa-30) mit Halogenierungsmitteln, wie z.B. Thionylchlorid, Phosphortribromid, Phosphortrichlorid oder Phosphor(V)-chlorid, gegebenenfalls in Gegenwart eines Verdünnungsmittels, bzw. mit Sulfonylierungsmitteln, wie z.B. Methansulfonsäurechlorid oder p-Toluolsulfonsäurechlorid, gegebenenfalls in Gegenwart eines Verdünnungsmittels und einer Base, umsetzt.

Als Verdünnungsmittel kommen bei der Halogenierung beispielsweise aromatische Kohlenwasserstoffe wie z.B. Benzol, Toluol, Xylol, und halogenierte Kohlenwasserstoffe wie z.B. Methylenchlorid, Chloroform, Tetrachlorkohlenstoff und Dichlorethan in Frage. Das Halogenierungsmittel kann in einem bis zu fünffachen Überschuß eingesetzt werden.

Die Reaktionstemperatur kann bei der Halogenierung in einem größeren Bereich variiert werden. Im allgemeinen wird die Reaktion zwischen 0°C und dem Siedepunkt des Verdünnungsmittels durchgeführt.

Als Verdünnungsmittel kommen bei der Sulfonylierung neben den oben als für die Halogenierungsreaktion geeignet beschriebenen auch noch beispielsweise Ether wie z.B. Diethylether oder Tetrahydrofuran in Frage.

Als Base werden bei der Sulfonylierung bevorzugt organische Basen wie z.B. Triethylamin, N,N-Dimethylanilin, Pyridin und 4-N,N-Dimethylaminopyridin eingesetzt.

Pro Mol der zu sulfonylierenden Verbindung werden im allgemeinen 0,8 bis 1,5 mol als Base eingesetzt.

Das Sulfonylierungsmittel wird im allgemeinen in mindestens äquimolaren Mengen eingesetzt.

Das erfindungsgemäße Verfahren (c) wird in Gegenwart einer Base durchgeführt. Es kommen hierbei alle üblichen anorganischen oder organischen Basen in Betracht. Vorzugsweise verwendbar sind Erdalkali- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (c) in einem größeren Bereich variiert werden. Im allgemeinen arbeitet man bei Temperaturen zwischen -20°C und + 150°C, vorzugsweise bei Temperaturen zwischen 0°C und 100°C.

Das erfindungsgemäße Verfahren (c) wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens (c) zur Herstellung der Verbindungen der Formel (Ia-30) setzt man pro Mol an Amid-Derivat der Formel (Va-30) im allgemeinen 1 bis 3 Mol, vorzugsweise 1,0 bis 1,5 Mol einer Base ein.

In einer bevorzugten Ausführungsform des erfindungsgemäßen Verfahrens (c) werden das Amid-Derivat der Formel (Va-30) und eine Base in einem geeigneten Verdünnungsmittel vermischt; das Gemisch wird bei der erforderlichen Temperatur bis zum Ende der Umsetzung gerührt und anschließend auf übliche Weise aufgearbeitet.

Das erfindungsgemäße Verfahren (d) wird unter Verwendung eines thienylierenden Mittels durchgeführt. Als solche kommen die üblichen zur Umwandlung von organischen Sauerstoffverbindungen in entsprechende Schwefelverbindungen geeigneten Reagentien in Betracht. Vorzugsweise verwendbar sind Phosphor(V)-sulfid, Hydrogensulfid und dessen Alkalimetallsalze sowie das sogenannte Lawesson-Reagenz.

Die Reaktionstemperaturen können bei der Durchführung des erfindungsgemäßen Verfahrens (d) in einem größeren Bereich variiert werden. Im allgemeinen arbeitet man bei Temperaturen zwischen -20°C und +150°C, vorzugsweise bei Temperaturen zwischen 0°C und 100°C.

Das erfindungsgemäße Verfahren (d) wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens (d) zur Herstellung der Verbindungen der Formel (Ia-30) setzt man pro Mol an Amidalkohol der Formel (IVa-30) im allgemeinen 1 bis 5 Mol, vorzugsweise 1,0 bis 2,5 Mol eines thienylierenden Mittels ein.

In einer bevorzugten Ausführungsform des erfindungsgemäßen Verfahrens (d) wird der Amidalkohol der Formel (IVa-30) in einem Verdünnungsmittel vorgelegt und das thienylierende Mittel wird dann eindosiert. Das Reaktionsgemisch wird dann bis zum Ende der Umsetzung bei der geeigneten Reaktionstemperatur gerührt und anschließend aufübliche Weise aufgearbeitet.

Die erfindungsgemäß verwendbaren Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp.

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis; Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Bruchidius obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella fit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Aus der Ordnung der Blattaria z.B. Periplaneta japonica, Periplaneta americana, Blattella germanica.

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp.

Weiter seien genannt Myzus persicae, Lipaphis erysimi, Aphis citrocola, Nippolachnus piri, Nezara antennata, Cletus punctiger, Riptortus clavatus;

Scirtothrips dorsalis, Thrips palmi, Ponticulothrips diospyrosi;
Oxya yezoensis, Locusta migratoria;
Anomala cuprea, Oulema oryzae, Lissorhoptrus oryzophilus Epilachna vigintioctomaculata;
Musea domestica, Culex pipiens;
Plutella xylostella, Spodoptera litura, Chilo suppressalis;
Tetranychus urticae, Tetranychus kanzawai, Panonychus ulmi, Panonychus citri, Dermatophagoides farinae, Tyrophagus putrescentiae, Polyphagotarsonemus latus, Omithonyssus bacoti, Ixodes ovatus, Haemaphysalis longicornis;
Pthirus pubis;
Pulex irritans, Ctenocephalides canis;
Peticulitermes speratus, Coptotermes formosanus;

Die Wirkstoffe können in die üblichen Formulierungen übergeführt werden, wie Lösungen, Emulsionen, Spritzpulver, Suspensionen, Pulver, Stäubemittel, fließfähige Mittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen und in Hüllmassen für Saatgut, ferner in Formulierungen mit Brennsätzen, wie Räucherpatronen, -dosen, -spiralen, Giftködern u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln und/oder Stabilisatoren.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, Trimethylbenzol oder Alkylnaphthaline, chlorierte Aromaten oder chlorierte aliphatische Kohlenwasserstoffe, wie Chlorbenzole, Dichlorethan, Trichlorethan, Chlorethylene oder Methylenchlorid, aliphatische Kohlenwasserstoffe, wie Cyclohexan, Hexan oder Paraffine, z.B. Erdölfraktionen, mineralische und pflanzliche Öle, wie Sojabohnen- und Olivenöl, Alkohole, wie Butanol, Methanol, Phenoxyethanol oder Glycol sowie deren Ether und Ester, Ketone, wie Aceton, Methylethylketon, Methylisobutylketon oder. Cyclohexanon, Ether wie Diethylether, Tetrahydrofuran, Dioxan, Nitrile wie Acetonitril, Ester wie Essigsäureethylester, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgae, wie Halogenkohlenwasserstoffe sowie Butan, Propan, Stickstoff und Kohlendioxid; als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Stärken, Sägemehl, Sojabohnenmehl, Fischmehl, Weizenmehl, Kokosnußschalen, Maiskolben und Tabakstengel; als Emulgier- und/oder schaumerzeugende Mittel kommen in Frage: z.B. nichtionogene und anionische Emulgatoren, wie Polyoxyalkylenalkylether, Polyoxyalkylenalkylarylether, Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate, Alkylarylsulfonate, Polyoxyalkylenalkylarylsulfonate sowie Eiweißhydrolysate; als Dispergiermittel und Haftmittel kommen in Frage: z.B. Lignin-Sulfitablaugen, Methylcellulose, Naphthalinsulfonsäure-Formalin-Kondensate, Stärken, Montmorillonit, synthetische wasserlösliche Makromoleküle Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Als Stabilisatoren seien z.B. Phosphorsäureester, Glykole, nichtionische oberflächenaktive Mittel, aromatische Diamine, pflanzliche Öle und epoxidierte Öle genannt.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,01 und 95 Gewichtsprozent Wirkstoff, vorzugsweise zwischen 0,5 und 90 %.

Diese Werte können auch je nach der verwendeten Formulierung in einem größeren Bereich schwanken. Beispielsweise können die Formulierungen im Fall von Emulsionen, Spritzpulvern, fließfähigen Mitteln etc, die Wirkstoffe in einer Konzentration von 0,01 bis 50 Gew.-%, bevorzugt von 0,1 bis 20 Gew.-% enthalten und im Fall von Pulvern, Granulaten etc. in einer Konzentration von 0,01 bis 20 Gew.-%, bevorzugt von 0,1 bis 10 Gew.-%.

Die Aufwandmenge der Verbindungen der Formel (Ia-30) hängt ab von der Art des Wirkstoffs, der Anwendungsform und der Größe des Schädlingsbefalls.

In der Landwirtschaft verwendet man im allgemeinen 1 bis 10 000 g, bevorzugt 10 bis 1 000 g pro Hektar. Im Fall der oben genannten Emulsionen, Spritzpulvern, fließfähigen Mittel etc. kann üblicherweise 1 000 bis 10 000-fach verdünnt werden und in einer Menge von 1 000 bis 10 000 Litern pro Hektar angewendet werden.

Im Fall der Pulver, Granulate etc. verwendet man im allgemeinen 2 bis 40 kg pro Hektar.

Aufdem Hygienesektor und gegen Insekten verwendet man die Mittel derart, daß pro $m^2$ 0,005 bis 100 g, bevorzugt 0,01 bis 50 g an Wirkstoffausgebracht werden.

Die erfindungsgemäßen Wirkstoffe können in ihren handelsüblichen Formulierungen sowie in den aus den Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Genannt seien die folgenden Verbindungen:

Acrinathrin, Alphamethrin, Betacyfluthrin, Bifenthrin, Brofenprox, Cis-Resmethrin, Clocythrin, Cycloprothrin, Cyfluthrin, Cyhalothrin, Cypermethrin, Deltamethrin, Esfenvalerate, Etofenprox, Fenpropathrin, Fenvalerate, Flucythrinate, Fluvalinate, Lambda-Cyhalothrin, Permethrin, Pyresmethrin, Pyrethrum, Silafluofen, Tralomethrin, Zetamethrin,
Alanycarb, Bendiocarb, Benfuracarb, Bufencarb, Butocarboxim, Carbaryl, Cartap, Ethiofencarb, Fenobucarb, Fenoxycarb, Isoprocarb, Methiocarb, Methomyl, Metolcarb, Oxamyl, Pirimicarb, Promecarb, Propoxur, Terbam, Thiodicarb, Thiofanox, Trimethacarb, XMC, Xylylcarb,
Acephate, Azinphos A, Azinphos M, Bromophos A, Cadusafos, Carbophenothion, Chlorfenvinphos, Chlormephos, Chlorpyrifos, Chlorpyrifos M, Cyanophos. Demeton M, Demeton-S-methyl, Demeton S, Diazinon, Dichlorvos, Dicliphos, Dichlorfenthion, Dicrotophos, Dimethoate, Dimethylvinphos, Dioxathion, Disulfoton, Edifenphos, Ethion, Etrimphos, Fenitrothion, Fenthion, Fonophos, Formothion, Heptenophos, Iprobenfos, Isazophos, Isoxathion, Phorate, Malathion, Mecarbam, Mervinphos, Mesulfenphos, Methacrifos, Methamidophos, Naled, Omethoate, Oxydemeton M, Oxydeprofos, Parathion A, Parathion M, Phenthoate, Phorate, Phosalone, Phosmet, Phosphamdon, Phoxim, Pirimiphos A, Pirimiphos M, Propaphos, Prothiophos, Prothoate, Pyraclophos, Pyridaphenthion, Quinalphos, Salithion, Sebufos, Sulfotep, Sulprofos, Tetrachlorvinphos, Temephos, Thiomethon, Thionazin, Trichlorfon, Triazophos, Vamidothion, DMTP (0,0-Dimethyl S-[5-methoxy-1,3,4-thiadiazol-2(3H)-onyl-(3)-methyl]-dithiophosphat), DDVP (Dimethyl 2,2-dichlorovinyl phosphat), CYAP (0,0-Dimethyl-0-4-cyanophenyl thiophosphat), Buprofezin, Chlorfluazuron, Diflubenzuron, Flucycloxuron, Flufenoxuron, Hexaflumuron, Pyriproxifen, Tebufenozide, Teflubenzuron, Triflumuron,
Imidacloprid, Nitenpyram, N-[(6-Chloro-3-pyridinyl)methyl]-N'-cyano-N-methylethanimidamid (NI-25),
Abamectin, Amitrazin, Avermectin, Azadirachtin, Bensultap, Bacillus thuringiensis, Cyromazine, Diafenthiuron, Emamectin, Ethofenprox, Fenpyrad, Fipronil, Flufenprox, Lufenuron, Metaldehyd, Milbemectin, Pymetrozine, Tebufenpyrad, Triazuron,
Aldicarb, Bendiocarb, Benfuracarb, Carbofuran, Carbosulfan, Chlorethoxyfos, Cloethocarb, Disulfoton, Ethophrophos, Etrimphos, Fenamiphos, Fipronil, Fonofos, Fosthiazate, Furathiocarb, HCH, Isazophos, Isofenphos, Methiocarb, Monocrotophos, Nitenpyram, Oxamyl, Phorate, Phoxim, Prothiofos, Pyrachlofos, Sebufos, Silafluofen, Tebupirimphos, Tefluthrin, Terbufos, Thiodicarb, Thiafenox,
Azocyclotin, Butylpyridaben, Clofentezine, Cyhexatin, Diafenthiuron, Diethion, Emamectin, Fenazaquin, Fenbutatin Oxide, Fenothiocarb, Fenpropathrin, Fenpyrad, Fenpyroximate, Fluazinam, Fluazuron, Flucycloxuron, Flufenoxuron, Fluvalinate, Fubfenprox, Hexythiazox, Ivemectin, Methidathion, Monocrotophos, Moxidectin, Naled, Phosalone, Profenofos, Pyraclofos, Pyridaben, Pyrimidifen, Tebufenpyrad, Thuringiensin, Triarathene sowie 4-Bromo-2-(4-chlorophenyl)-1-(ethoxymethyl)-5-(trifluoromethyl)-1H-pyrrole-3-carbonitril (AC 303630),
Dicotol, Chlorobenzilate, Bromopropylate, Chlorofenson, BPPS.

Besonders günstige Mischpartner sind weiterhin z.B. die folgenden:

**Fungizide:**

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid; (E)-2-Methoxyimino-N-methyl-2-(2-phenoxyphenyl)-acetamid; 8-Hydroxyquinolinsulfat; Methyl-(E)-2-{2-[6-(2-cyanophenoxy)-pyrimidin-4-yloxy]-phenyl}-3-methoxyacrylat; Methyl-(E)-methoximino-[alpha-(o-tolyloxy)-o-tolyl]acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropierin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan,
Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil,
Nickel-dimethyldithiocarbamat, Nitrothal-isopropyl, Nuarimol,

Ofurace, Oxadixyl, Oxamocarb, Oxycarboxin,
Pefurazoat, Penconazol, Pencycuron, Phosdiphen, Phthalid, Pimaricin, Piperalin, Polycarbamate, Polyoxin, Probenazol, Prochloraz, Procymidon, Propamocarb, Propiconazole, Propineb, Pyrazophos, Pyrifenox, Pyrimethanil, Pyroquilon, Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinelozolin,
Zineb, Ziram

**Bakterizide**:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
Als Lockstoffe seien z.B. Benzoesäure, 4-Allyl-2-methoxyphenol und 4-(p-Acetoxyphenyl)-2-butanon genannt.
Die erfindungsgemäß verwendbaren Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv sein muß.
Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.
Die Anwendung geschieht in einer den Anwendungsformen angepaßten üblichen Weise.
Die erfindungsgemäß verwendbaren Wirkstoffe wirken nicht nur gegen Pflanzen-, Hygiene- und Vorratsschädlinge, sondern auch auf dem veterinärmedizinischen Sektor gegen tierische Parasiten (Ektoparasiten und Endoparasiten) wie Schildzecken, Lederzecken, Räudemilben, Laufmilben, Fliegen (stechend und leckend), parasitierende Fliegenlarven, Läuse, Haarlinge, Federlinge, Flöhe und endoparasitisch lebende Würmer. Beispielsweise zeigen sie eine hervorragende Wirksamkeit gegen Zecken, wie beispielsweise Boophilus microplus.
Die erfindungsgemäß verwendbaren Wirkstoffe der Formel (Ia-30) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.
Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.
Bei der Anwendung für Vieh, Geflügel, Haustiere etc. kann man die Wirkstoffe der Formel (Ia-30) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.
Die Herstellung und die Verwendung der erfindungsgemäßen Stoffe wird durch die folgenden Beispiele veranschaulicht.

**Herstellungsbeispiele**

**Beispiel 24**

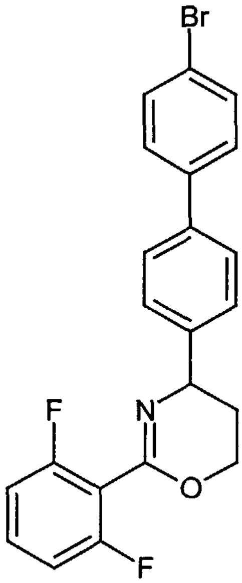

Fp. 134 bis 138°C,
sowie die Verbindungen der Formel (Ia-30)

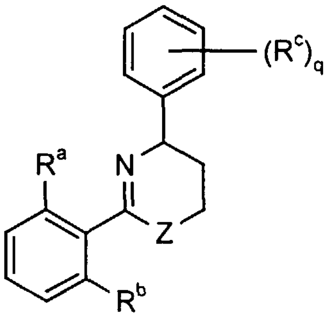

| Bsp.-Nr. | Z | $R^a$ | $R^b$ | $(R^c)_q$ | Fp. oder $n_D^{25}$ |
|---|---|---|---|---|---|
| 306 | O | F | F | 3-$Si(CH_3)_3$ | 1.5396 |
| 307 | O | F | F | 4-$Si(CH_3)_3$ | 1.5441 |
| 308 | O | Cl | F | 4-$Si(CH_3)_3$ | 1.5552 |
| 309 | O | F | F | 4-$Si(CH_2CH_3)_3$ | 1.5440 |
| 310 | O | Cl | F | 4-$Si(CH_2CH_3)_3$ | 1.5493 |
| 311 | O | F | F | 4-$Si[C(CH_3)_3, (CH_3)_2]$ | 1.5410 |
| 314 | O | F | F | 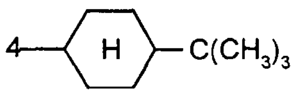 | 1.5421 |
| 315 | O | Cl | F | 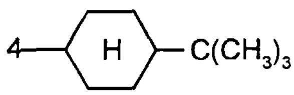 | 1.5516 |
| 317 | O | F | F | 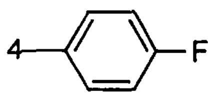 | 1.5954 |
| 318 | O | Cl | F | 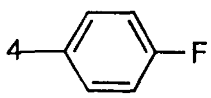 | 1.6052 |
| 319 | O | F | F | 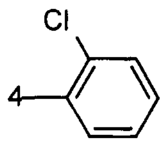 | 1.6116 |
| 320 | O | F | F | 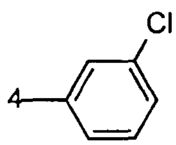 | 1.6187 |

| Bsp.-Nr. | Z | $R^a$ | $R^b$ | $(R^c)_q$ | Fp. oder $n_D^{25}$ |
|---|---|---|---|---|---|
| 321 | O | F | F | 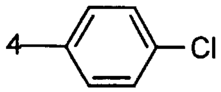 | 126-129°C |
| 322 | O | F | F | 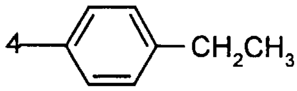 | 1.6126 |
| 323 | O | F | F | 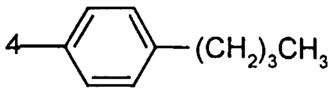 | 98-99°C |
| 324 | O | F | F | 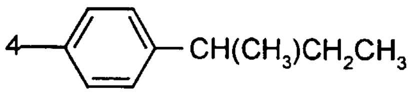 | 1.5942 |
| 325 | O | F | F | 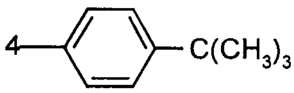 | 1.5476 |
| 326 | O | F | F | 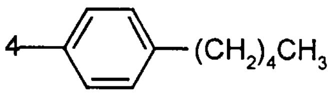 | 68-69°C |
| 327 | O | F | F | 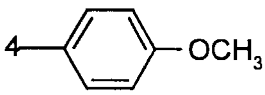 | 124-125°C |
| 328 | O | F | F | 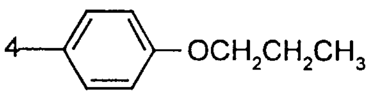 | 1.5962 |
| 329 | O | F | F | 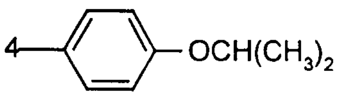 | 1.5755 |
| 330 | O | F | F | 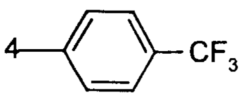 | 108-113°C |
| 331 | O | F | Cl | 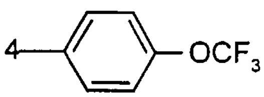 | 1.5804 |
| 332 | O | F | F | 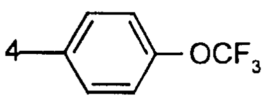 | 105-106°C |

| Bsp.-Nr. | Z | $R^a$ | $R^b$ | $(R^c)_q$ | Fp. oder $n_D^{25}$ |
|---|---|---|---|---|---|
| 333 | O | F | F | 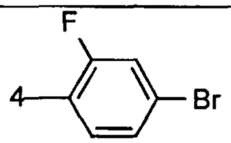 | 101-103°C |
| 334 | O | Cl | F | 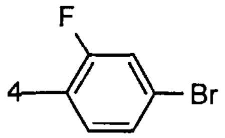 | 1.6113 |
| 335 | O | F | F | 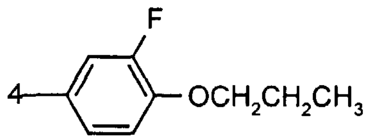 | 1.5922 |
| 336 | O | F | F | 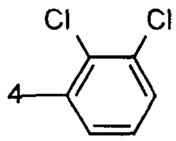 | 1.6154 |
| 337 | O | H | Cl | 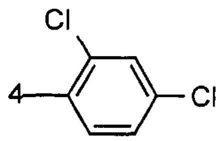 | 1.6461 |
| 338 | O | F | F | 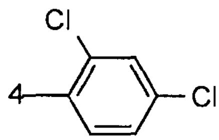 | 117-119°C |
| 339 | O | F | F | 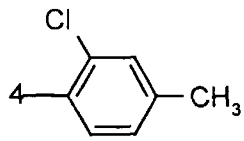 | 1.6082 |
| 340 | O | F | F | 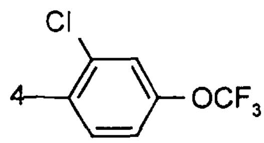 | 1.5695 |
| 341 | O | F | F | 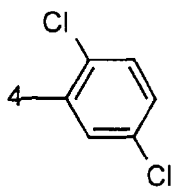 | 1.6114 |

| Bsp.-Nr. | Z | $R^a$ | $R^b$ | $(R^c)_q$ | Fp. oder $n_D^{25}$ |
|---|---|---|---|---|---|
| 342 | O | F | F | 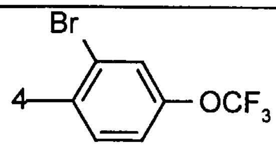 | 1.5856 |
| 343 | O | F | F | 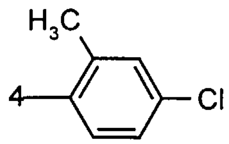 | 1.6071 |
| 344 | O | F | F | 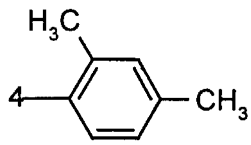 | 1.5961 |
| 345 | O | F | F | 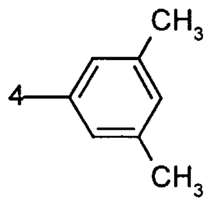 | 1.6068 |
| 346 | O | F | F | 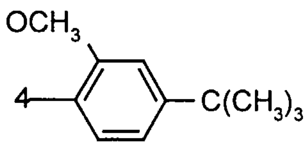 | 1.5830 |
| 347 | O | F | F | 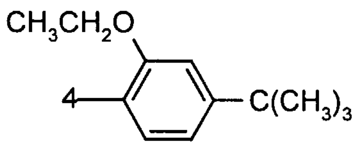 | 1.5804 |
| 348 | O | F | F | 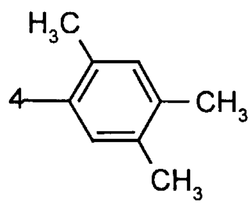 | 1.5946 |
| 349 | O | F | F | 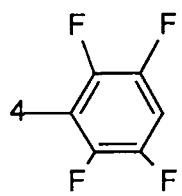 | 1.5631 |

| Bsp.-Nr. | Z | $R^a$ | $R^b$ | $(R^c)_q$ | Fp. oder $n_D^{25}$ |
|---|---|---|---|---|---|
| 350 | O | Cl | F | 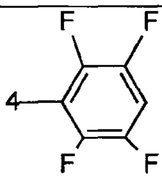 | 1.5718 |
| 351 | O | Cl | F | 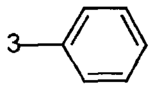 , 4-$O(CH_2)_3CH_3$ | 1.5963 |
| 352 | O | F | F | 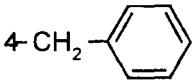 | 1.5894 |
| 353 | O | F | F | 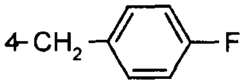 | 1.5763 |
| 354 | O | Cl | F | 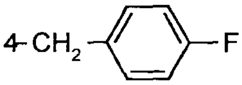 | 1.5878 |
| 355 | O | F | F | 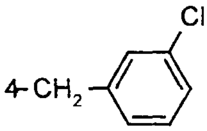 | 1.5954 |
| 356 | O | F | F | 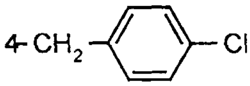 | 1.5928 |
| 357 | O | F | F | 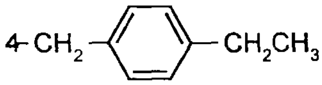 | 1.5800 |
| 358 | O | F | F | 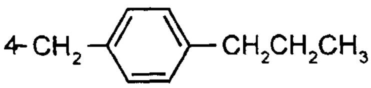 | 1.5741 |
| 359 | O | F | F | 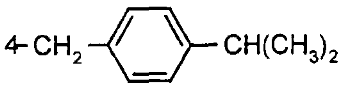 | 1.5821 |
| 360 | O | F | F | 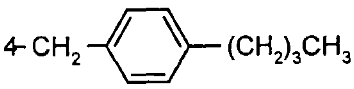 | 1.5688 |
| 361 | O | Cl | F | 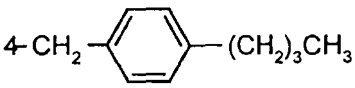 | 1.5753 |

| Bsp.-Nr. | Z | $R^a$ | $R^b$ | $(R^c)_q$ | Fp. oder $n_D^{25}$ |
|---|---|---|---|---|---|
| 362 | O | F | F | 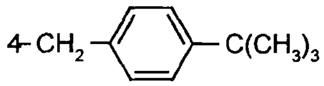 | 1.5738 |
| 363 | O | F | F | 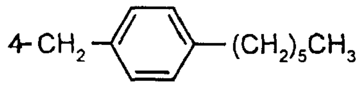 | 1.5586 |
| 364 | O | F | F | 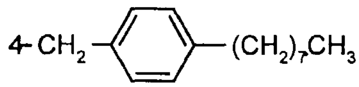 | 1.5530 |
| 365 | O | F | F | 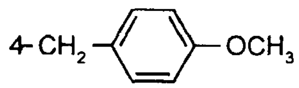 | 1.5846 |
| 366 | O | F | F | 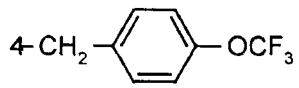 | 1.5613 |
| 367 | O | Cl | F | 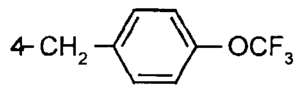 | 1.5714 |
| 368 | O | F | F | 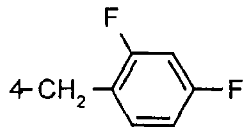 | 1.5692 |
| 369 | O | F | F | 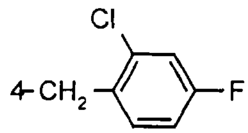 | 1.5896 |
| 370 | O | F | F | 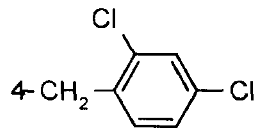 | 1.5978 |
| 371 | O | F | F | 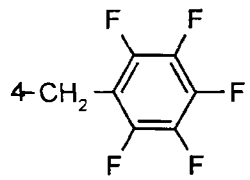 | 1.5491 |
| 372 | O | F | F | 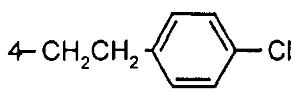 | 1.5867 |
| 373 | O | F | F | 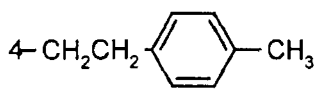 | 1.5818 |

| Bsp.-Nr. | Z | $R^a$ | $R^b$ | $(R^c)_q$ | Fp. oder $n_D^{25}$ |
|---|---|---|---|---|---|
| 374 | O | F | F | 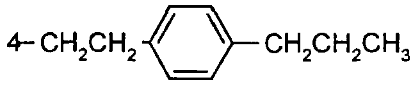 | 1.5671 |
| 375 | O | F | F | 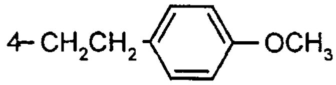 | 72-72,5°C |
| 376 | O | F | F | 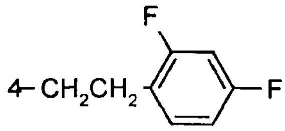 | 1.5662 |
| 377 | O | F | F | 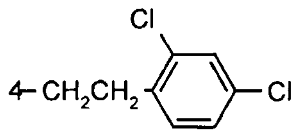 | 1.5904 |
| 378 | O | F | F | 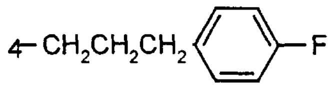 | 1.5654 |
| 379 | O | F | F | 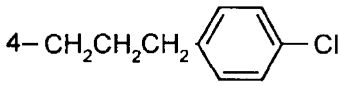 | 1.5813 |
| 380 | O | Cl | F | 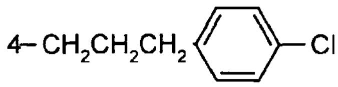 | 1.5911 |
| 382 | O | F | F | 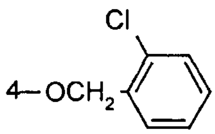 | 1.5940 |
| 383 | O | F | F | 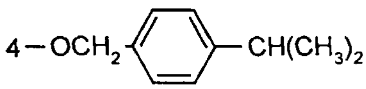 | 1.5746 |
| 384 | O | F | F | 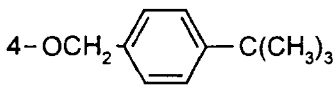 | 1.5671 |
| 385 | O | F | F | 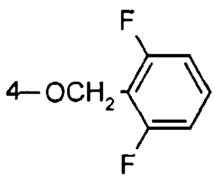 | 1.5651 |
| 406 | O | F | F | 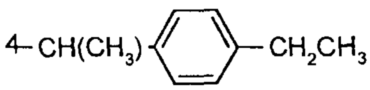 | 1.5712 |

| Bsp.-Nr. | Z | $R^a$ | $R^b$ | $(R^c)_q$ | Fp. oder $n_D^{25}$ |
|---|---|---|---|---|---|
| 407 | O | F | F | 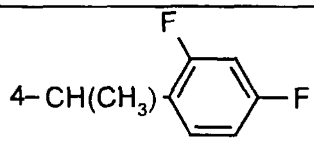 | 1.5654 |
| 408 | O | Cl | F | 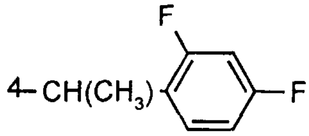 | 1.5750 |
| 409 | O | F | F | 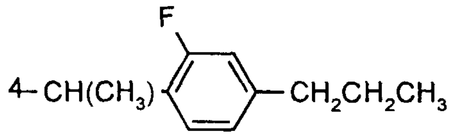 | 1.5694 |
| 410 | O | F | F | 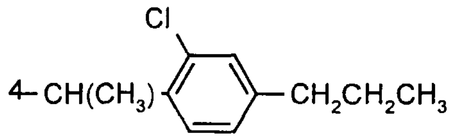 | 1.5797 |
| 411 | O | F | F | 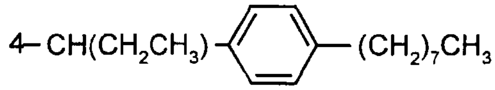 | 1.5428 |
| 412 | O | F | F | 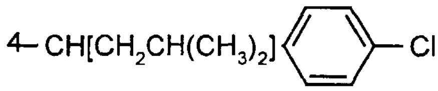 | 1.5773 |
| 413 | O | F | F | 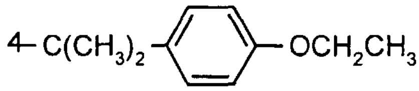 | 1.5698 |
| 414 | O | F | F | 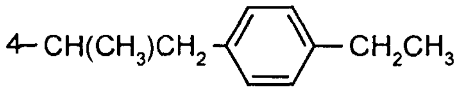 | 1.5687 |
| 415 | O | F | F | 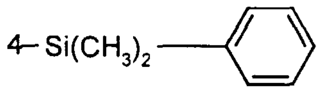 | 1.6792 |
| 416 | O | Cl | F | 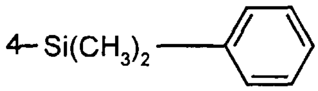 | 1.5892 |
| 445 | O | F | F | 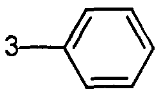 | 119~120°C |

| Bsp.-Nr. | Z | $R^a$ | $R^b$ | $(R^c)_q$ | Fp. oder $n_D^{25}$ |
|---|---|---|---|---|---|
| 446 | O | Cl | F | 3 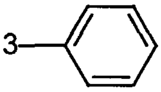 | 113~115°C |

| Bsp.Nr. | Verbindung | physik. Konstante |
|---|---|---|
| 470 | 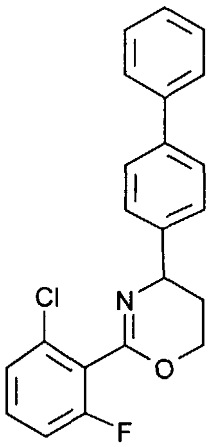 | Fp. 96-98°C |

* δ [ppm] = chemische Verschiebung in ppm; $^1$H-NMR-Spektren gemessen in $CDCl_3$ mit TMS (Tetramethylsilan) als Standard, Zuordnung der Protonen jeweils wie in der Formel Beispiel 25

** MS = Massenspektrum

*** log P = negativer dekadischer Logarithmus des Alkan/Wasser Verteilungskoeffizienten, bestimmt durch HPLC-Analytik mit $H_2O/CH_3CN$ als Laufmittel auf 125 x 4,0 mm Kromasil 120 C 18 (5μm); Fluß: 1,5 ml/min.

Im folgenden wird die Herstellung einzelner in der Tabelle genannter Beispiele beschrieben:

Formulierungsbeispiele (Teile = Gewichtsteile)

Formulierungsbeispiel 1 (Emulsion)

10 Teile einer erfindungsgemäßen Verbindung, 5 Teile Alkyl-arylsulfonat, 5 Teile Polyoxyalkylenalkyl-arylether

und 80 Teile Xylol werden gleichmäßig zu einer Emulsion vermischt.

Formulierungsbeispiel 6 (fließfähiges Mittel)

10 Teile der Verbindung gemäß Beispiel 445, 2 Teile Alkylbenzolsulfonatsalz, 3 Teile Polyoxyalkylenalkyl-arylether, 5 Teile Propylenglykol und 79 Teile Wasser werden zu einer einheitlichen Dispersion gerührt, 1 Teil eines Antischaummittels wird zugegeben und die Mischung einheitlich gemahlen.

Biologische Beispiele

Die in den Versuchen eingesetzte Vergleichssubstanz 2,4-Diphenyl-2-oxazin ist aus Zh. Org. Khim. 18, 178 (1982) bekannt.

Beispiel: C

**Plutella-Test**

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe *Plutella maculipennis* besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigt z.B. die Verbindung des Herstellungsbeispiels 24 bei einer Wirkstoffkonzentration von 0,1% nach 7 Tagen einen Abtötungsgrad von 80 bis 100%.

Beispiel F

**Phaedon-Larven-Test**

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschten Konzentrationen.

Kohlblätter (Brassica oleracea) werden durch Tauchen in die Wirkstoffzubereitung der gewünschten Konzentration behandelt und mit Meerrettichblattköfer-Larven (Phaedon cochleariae) besetzt, solange die Blätter noch feucht sind.

Nach der gewünschten Zeit wird die Wirkung in % bestimmt. Dabei bedeutet 100%, daß alle Käfer-Larven abgetötet wurden; 0% bedeutet, daß keine Käfer-Larven abgetötet wurden.

In diesem Test zeigte z.B. die Verbindung gemäß Herstellungsbeispiel 24 bei einer beispielhaften Wirkstoffkonzentration von 0,1 % einen Abtötungsgrad von 100 % nach 7 Tagen.

Beispiel G

**Spodoptera-Test**

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschten Konzentrationen.

Kohlblätter (Brassica oleracea) werden durch Tauchen in die Wirkstoffzubereitung der gewünschten Konzentration behandelt und mit Raupen des Eulenfalters Spodoptera frugiperda) besetzt, solange die Blätter noch feucht sind.

Nach der gewünschten Zeit wird die Wirkung in % bestimmt. Dabei bedeutet 100 %, daß alle Raupen abgetötet wurden; 0 % bedeutet, daß keine Raupen abgetötet wurden.

In diesem Test bewirkte z.B. die Verbindung gemäß Herstellungsbeispiel 24 bei einer beispielhaften Wirkstoffkonzentration von 0,1 % eine Abtötung von 100 % nach 7 Tagen.

Beispiel H

**Tetranychus Test** (ovicide Wirkung)

In den Deckel eines mit etwas Wasser gefüllten Eiscrembechers (Durchmesser 9 cm) wird ein Loch gebohrt und durch dieses ein Filterpapier in Form eines Streifens eingeführt, welches sich mit dem Wasser vollsaugt. Darauf wird das Blatt einer Kidney-Bohne gelegt.

10 weibliche Insekten von Tetranychus urticae werden auf das Blau gebracht, dort für 24 Stunden zur Eiablage belassen und dann entfernt.

Die mit Wasser auf eine Wirkstoffkonzentration von 500 ppm verdünnte Emulsion des Formulierungsbeispiels 1 wird aufgebracht und der Behälter bei einer Temperatur von 25°C stehengelassen. Nach 7 Tagen wird die Zahl der geschlüpften Larven unter dem Mikroskop bestimmt und die ovicide Wirkung nach der unten angegebenen Gleichung bestimmt. Der Test wurde dreimal wiederholt. 2,4-Diphenyl-2-oxazin wurde als Vergleichssubstanz eingesetzt.

$$\text{Ovicide Wirkung (\%)} = \frac{\text{Zahl der gelegten Eier - Zahl der geschlüpften Larven}}{\text{Zahl der gelegten Eier}} \times 100$$

Die Verbindungen gemäß den einzelnen Herstellungsbeispielen zeigten die jeweils angegebenen ovicide Wirkung.

| | | | | |
|---|---|---|---|---|
| 352 = 88 % | 353 = 90 % | 354 = 90 % | 355 = 92 % | 357 = 95 % |
| 365 = 93 % | 366 = 90 % | 367 = 93 % | 368 = 98 % | 370 = 95 % |
| 371 = 90 % | 373 = 90 % | 374 = 83 % | 375 = 78 % | 376 = 93 % |
| 377 = 90 % | 378 = 95 % | 380 = 98 % | 385 = 90 % | 412 = 93 % |
| 413 = 78 % | 414 = 93 % | 370 = 85 % | | |

Alle anderen Verbindungen gemäß den Herstellungsbeispielen zeigten in diesem Test 100 % ovicide Wirkung. Die Vergleichssubstanz zeigte lediglich 30 % ovicide Wirkung.

Beispiel I

**Tetranychus Test** (ovicide Wirkung)

In den Deckel eines mit etwas Wasser gefüllten Eiscrembechers (Durchmesser 9 cm) wird ein Loch gebohrt und durch dieses ein Filterpapier in Form eines Streifens eingeführt, welches sich mit dem Wasser vollsaugt. Darauf wird das Blau einer Kidney-Bohne gelegt.

10 weibliche Insekten von Tetranychus kanzawai werden auf das Blau gebracht, dort für 24 Stunden zur Eiablage belassen und dann entfernt.

Die mit Wasser auf eine Wirkstoffkonzentration von 500 ppm verdünnte Emulsion des Formulierungsbeispiels 1 wird aufgebracht und der Behälter bei einer Temperatur von 25°C stehengelassen. Nach 7 Tagen wird die Zahl der geschlüpften Larven unter dem Mikroskop bestimmt und die ovicide Wirkung wie bei Beispiel H bestimmt. Der Test wurde dreimal wiederholt. 2,4-Diphenyl-2-oxazin wurde als Vergleichssubstanz eingesetzt.

Die Verbindungen gemäß den einzelnen Herstellungsbeispielen zeigten die jeweils angegebenen ovicide Wirkung.

| | | | | |
|---|---|---|---|---|
| 332 = 98 % | 352 = 95 % | 353 = 90 % | 354 = 93 % | 355 = 90 % |
| 365 = 85 % | 368 = 98 % | 371 = 85 % | 373 = 93 % | 376 = 90 % |
| 375 = 75 % | 378 = 85 % | 385 = 98 % | 413 = 75 % | |

Alle anderen Verbindungen gemäß den Herstellungsbeispielen zeigten in diesem Test 100 % ovicide Wirkung. Die Vergleichssubstanz zeigte lediglich 25 % ovicide Wirkung.

Beispiel K

**Tetranychus Test** (ovicide Wirkung)

In den Deckel eines mit etwas Wasser gefüllten Eiscrembechers (Durchmesser 9 cm) wird ein Loch gebohrt und durch dieses ein Filterpapier in Form eines Streifens eingeführt, welches sich mit dem Wasser vollsaugt. Darauf wird das Blatt einer Kidney-Bohne gelegt.

10 weibliche Insekten von Tetranychus uhicae werden auf das Blau gebracht, dort für 24 Stunden zur Eiablage belassen und dann entfernt.

Nach 8 Tagen bei einer konstanten Temperatur von 25°C werden die Protonymphen gezählt, die mit Wasser auf eine Wirkstoffkonzentration von 500 ppm verdünnte Emulsion des Formulierungsbeispiels 1 wird aufgebracht und der Behälter bei einer Temperatur von 25°C stehengelassen. Nach 7 Tagen wird die Zahl der Insekten unter dem Mikroskop bestimmt und die abtötende Wirkung nach folgender Gleichung bestimmt. Der Test wurde dreimal wiederholt. 2,4-Diphenyl-2-oxazin wurde als Vergleichssubstanz eingesetzt.

$$\text{Abtötende Wirkung (\%)} = \frac{\text{Zahl der Protonymphen - Zahl Insekten}}{\text{Zahl der Protonymphen}} \times 100$$

Die Verbindungen gemäß den einzelnen Herstellungsbeispielen zeigten die jeweils angegebenen ovicide Wirkung.

| | | |
|---|---|---|
| 366 = 98 % | 375 = 90 % | 377 = 90 % |

Alle anderen Verbindungen gemäß den Herstellungsbeispielen zeigten in diesem Test 100 % abtötende Wirkung. Die Vergleichssubstanz zeigte lediglich 25 % abtötende Wirkung.

Beispiel L

**Myzus Test**

Sämlinge im 2-Blatt-Stadium des Japanischen Rettichs, gezogen in Schalen, werden mit je 5 weiblichen Insekten von Myzus persicae besetzt.

Die Insekten werden 3 Tage dort belassen, um Larven zu legen und dann entfernt.

Die mit Wasser auf eine Wirkstoffkonzentration von 500 ppm verdünnte Emulsion des Formulierungsbeispiels 1 wird aufgebracht und die so behandelten Sämlinge in ein Gewächshaus gestellt. Nach 96 Stunden wird die abtötende Wirkung in % bestimmt. Dabei bedeutet 100 %, daß alle Insekten abgetötet wurden, 0 % bedeutet, daß keine Insekten abgetötet wurden. Der Test wird dreimal wiederholt. 2,4-Diphenyl-2-oxazin wurde als Vergleichssubstanz eingesetzt.

Die Verbindungen gemäß den einzelnen Herstellungsbeispielen zeigten in diesem Test die jeweils angegebene abtötende Wirkung:

| | | | | |
|---|---|---|---|---|
| 323 = 79 % | 324 = 93 % | 326 = 75 % | 330 = 69 % | 331 = 82 % |
| 332 = 93 % | 333 = 61 % | 338 = 77 % | | |

100 % abtötende Wirkung zeigten die Verbindungen gemäß den Herstellungsbeispielen Nr. 307, 308, 309, 310, 317, 318, 319, 320, 321, 322, 325, 445, 446, die Vergleichssubstanz zeigte lediglich eine abtötende Wirkung von 13 %.

Beispiel M

**Aphis Test**

Gurken-Sämlinge im 1-Blatt-Stadium, gezogen in Schalen, werden mit je 5 weiblichen Insekten von Aphis gossypii besetzt. Die Insekten werden 3 Tage dort belassen, um Larven zu legen und dann entfernt.

Die mit Wasser auf eine Wirkstoffkonzentration von 500 ppm verdünnte Emulsion des Formulierungsbeispiels 1 wird aufgebracht und die so behandelten Sämlinge in ein Gewächshaus gestellt.

Nach 96 Stunden wird die abtötende Wirkung in % bestimmt.

Dabei bedeutet 100 %, daß alle Insekten abgetötet wurden, 0 % bedeutet, daß keine Insekten abgetötet wurden. Der Test wurde dreimal wiederholt. 2,4-Diphenyl-2-oxazin wurde als Vergleichssubstanz eingesetzt.

Die Verbindungen gemäß den einzelnen Herstellungsbeispielen zeigten in diesem Test die jeweils angegebene abtötende Wirkung.

| | | |
|---|---|---|
| 317 = 95 % | 319 = 90 % | 325 = 85 % |

100 % abtötende Wirkung zeigten die Verbindungen gemäß den Herstellungsbeispielen 307, 308, 309, 310, 318, 320, 321, 322, 323, 324, 326, 331, 332, 338, 445, 446,
die Vergleichssubstanz zeigte lediglich eine abtötende Wirkung von 6 %.

Beispiel N

**Nephotettix Test**

Die mit Wasser auf eine Wirkstoffkonzentration von 500 ppm verdünnte Emulsion des Formulierungsbeispiels 1 wird auf in Töpfen gezogene Reis-Sämlinge aufgebracht. Nach dem Trocknen wird ein Zylinder aus Acrylharz über jeden Topf gestülpt, jeder Sämling wird mit 10 Larven von Nephotettix cincticeps besetzt und der Topfmit Gaze abgedeckt.

Die so behandelten Sämlinge werden in ein Gewächshaus gestellt. Nach 7 Tagen wird die abtötende Wirkung bestimmt.

Dabei bedeutet 100 %, daß alle Larven abgetötet wurden, 0 % bedeutet, daß keine Larven abgetötet wurden.

Der Test wurde dreimal wiederholt. Als Vergleichssubstanz wurde 2,4-Diphenyl-2-oxazin eingesetzt.

Die Verbindungen gemäß den einzelnen Herstellungsbeispielen zeigten in diesem Test die jeweils angegebene abtötende Wirkung:

333 = 67 %

100 % abtötende Wirkung zeigten die Verbindungen gemäß den Herstellungsbeispielen 306, 445, 446, die Vergleichssubstanz zeigte keine abtötende Wirkung.

Beispiel O

**Nilaparvata Test**

Die mit Wasser auf eine Wirkstoffkonzentration von 500 ppm verdünnte Emulsion des Formulierungsbeispiels 1 wird auf in Töpfen gezogene Reis-Sämlinge aufgebracht. Nach dem Trocknen wird ein Zylinder aus Acrylharz über jeden Topf gestülpt, jeder Sämling wird mit 10 Larven von Nilaparvata lugens besetzt und der Topf mit Gaze abgedeckt.

Die so behandelten Sämlinge werden in ein Gewächshaus gestellt. Nach 7 Tagen wird die abtötende Wirkung bestimmt.

Dabei bedeutet 100 %, daß alle Larven abgetötet wurden, 0 % bedeutet, daß keine Larven abgetötet wurden. Der Test wurde dreimal wiederholt. Als Vergleichssubstanz wurde 2,4-Diphenyl-2-oxazin eingesetzt.

Die Verbindungen gemäß den einzelnen Herstellungsbeispielen zeigten in diesem Test die jeweils angegebene abtötende Wirkung:

100 % abtötende Wirkung zeigten die Verbindungen gemäß den Herstellungsbeispielen 306, 445, 446, die Vergleichssubstanz zeigte keine abtötende Wirkung.

Test P

**Plutella Test**

Sämlinge des Japanischen Rettichs im 2-Blatt-Stadium, gezogen in Töpfen, wurden mit je 15 Larven von Plutella xylostella besetzt. Die mit Wasser auf eine Wirkstoffkonzentration von 500 ppm verdünnte Emulsion des Formulierungsbeispiels 1 wird aufgebracht. Die so behandelten Sämlinge werden in ein Gewächshaus gebracht. Nach 3 Tagen wird die abtötende Wirkung in % bestimmt.

Dabei bedeutet 100 %, daß alle Larven abgetötet wurden, 0 % bedeutet, daß keine Larven abgetötet wurden. Der Test wurde dreimal wiederholt. Als Vergleichssubstanz wurde 2,4-Diphenyl-2-oxazin eingesetzt.

Die Verbindeungen gemäß den einzelnen Herstellungsbeispielen zeigten in diesem Test die jeweils angegebene abtötende Wirkung:

| | | | |
|---|---|---|---|
| 335 = 95 % | 336 = 92 % | 337 = 98 % | 345 = 92 % |

100 % abtötende Wirkung zeigten die Verbindungen gemäß den Herstellungsbeispielen 317 bis 326, 330 bis 334, 338 bis 344, 346, 347, 445 und 446, die Vergleichssubstanz zeigte keine abtötende Wirkung.

Test Q

**Culex Test**

Die Emulsion des Formulierungsbeispiels 1 wird mit Wasser auf eine Wirkstoffkonzentration von 500 ppm verdünnt. Je 50 ml dieser Zubereitung werden in 120 ml Eiscreme-Becher gefüllt. In jeden Becher gibt man 20 Larven von Culex pipiens und etwa 40 mg Hefe als Nahrung. Nach 7 Tagen wird die abtötende Wirkung in % bestimmt.

Dabei bedeutet 100 %, daß alle Larven abgetötet wurden, 0 % bedeutet, daß keine Larven abgetötet wurden. Der Test wurde dreimal wiederholt. 2,4-Diphenyl-2-oxazin wurde als Vergleichssubstanz eingesetzt.

Die Verbindungen gemäß den einzelnen Herstellungsbeispielen zeigten in diesem Test die jeweils angegebene abtötende Wirkung:

| | | | | |
|---|---|---|---|---|
| 327 = 78 % | 328 = 80 % | 329 = 80 % | 335 = 75 % | 365 = 80 % |
| 375 = 83 % | 413 = 78 % | | | |

Alle anderen Verbindungen gemäß den Herstellungsbeispielen zeigten eine abtötende Wirkung von 100 %. Die Vergleichssubstanz zeigte lediglich 6 % abtötende Wirkung.

Beispiel S

**Blowfly-Larven-Test**

Testtiere: Lucilia cuprina-Larven
Emulgator: 35 Gewichtsteile Ethylenglykolmonomethylether 35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Gemisches und verdünnt das so erhaltene Emulsionskonzentrat mit Wasser auf die jeweils gewünschte Konzentration.

Etwa 20 *Lucilia cuprina res*.-Larven werden in ein Teströhrchen gebracht, welches ca. 1 $cm^3$ Pferdefleisch und 0,5 ml der Wirkstoffzubereitung enthält. Nach 24 Stunden wird die Wirksamkeit der Wirkstoffzubereitung ermittelt. Dabei bedeutet 100 %, daß alle Blowfly-Larven abgetötet wurden; 0 % bedeutet, daß keine Blowfly-Larven abgetötet wurden.

In diesem Test bewirkten z.B. die Verbindung gemäß Herstellungsbeispielen 24 und 321 bei einer beispielhaften Wirkstoffkonzentration von 1000 ppm eine Abtötung von 100 %.

**Patentansprüche**

**1.** Azin-Derivate der Formel (Ia-30)

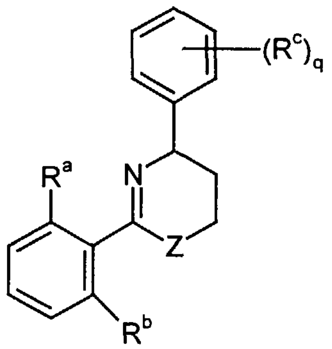

(Ia-30)

in welcher

$R^a$ für Wasserstoff, Fluor oder Chlor steht,

$R^b$ für Fluor oder Chlor steht,

q für eine Zahl von 1 bis 5 steht,

$R^c$ für Tri($C_1$-$C_6$ Alkyl)silyl, für gegebenenfalls einfach bis dreifach durch $C_1$-$C_6$ Alkyl substituiertes $C_3$-$C_7$-Cycloalkyl oder für

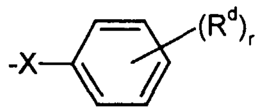

steht, worin

X für eine direkte Bindung, $C_1$-$C_6$ Alkandiyl, $C_1$-$C_6$-Oxy-Alkandiyl oder Di($C_1$-$C_6$ Alkyl)silyl steht, wobei

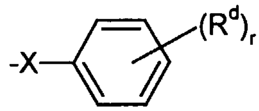

nicht für unsubstituiertes Phenylmethoxy steht,

r für eine Zahl von 0 bis 5 steht,

$R^d$ für $C_1$-$C_{12}$-Alkyl, $C_1$-$C_{12}$-Alkoxy, Halogen, $C_1$-$C_6$ Halogenalkyl, $C_1$-$C_6$ Halogenalkoxy oder Tri($C_1$-$C_6$ Alkyl)silyl steht und

Z für Sauerstoff oder Schwefel steht,
ausgenommen die Verbindung der Formel

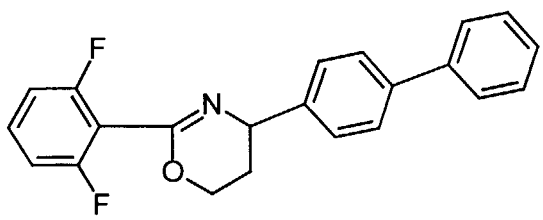

**2.** Verfahren zur Herstellung von Azin Derivaten der Formel (Ia-30) gemäß Anspruch 1, dadurch gekennzeichnet, daß man

a) Aminoalkohole der Formel

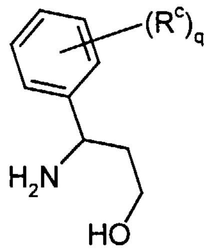

(II-30),

in welcher $R^c$ und q die in Anspruch 1 genannte Bedeutung haben, mit einer Carbonsäure der Formel

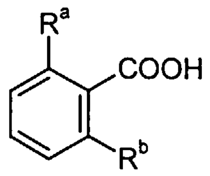

(III-30),

in welcher $R^a$ und $R^b$ die in Anspruch 1 genannten Bedeutungen haben, mit einem wasserentziehenden Mittel, gegebenenfalls in Gegenwart eines Verdünnungsmittels umsetzt; oder

b) Amidalkohole der Formel

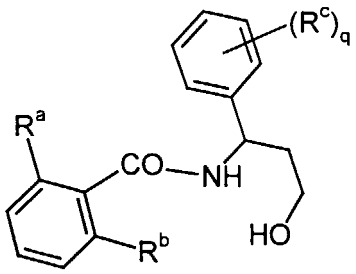

(IVa-30)

in welchen $R^a$, $R^b$, $R^c$ und q die in Anspruch 1 genannten Bedeutungen haben, mit einem wasserentziehenden Mittel, gegebenenfalls in Gegenwart eines Verdünnungsmittels umsetzt; oder

c) Amid-Derivate der Formel

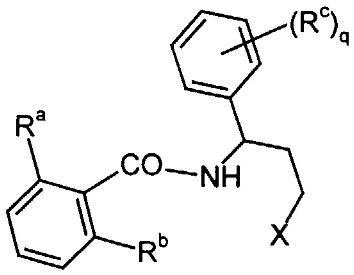

(Va-30)

in welchen $R^a$, $R^b$, $R^c$ und q die in Anspruch 1 genannten Bedeutungen haben, und X für Halogen, Alkylsulfonyloxy oder Arylsulfonyloxy steht, mit einer Base, gegebenenfalls in Gegenwart eines Verdünnungsmittels umsetzt;

oder

d) Amidalkohole der Formel

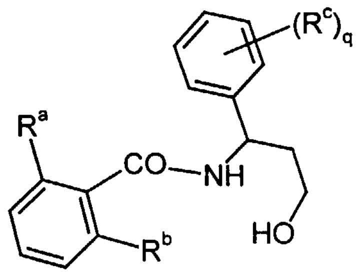

(IVa-30)

in welchen $R^a$, $R^b$, $R^c$ und q die in Anspruch 1 genannten Bedeutungen haben, mit thienylierenden Mitteln, gegebenenfalls in Gegenwart eines Verdünnungsmittels umsetzt.

**3.** Mittel zur Bekämpfung von tierischen Schädlingen, gekennzeichnet durch einen Gehalt an mindestens einem Azin-Derivat der Formel (Ia-30) gemäß Anspruch 1.

**4.** Verfahren zur Bekämpfung von tierischen Schädlingen, dadurch gekennzeichnet, daß man Azin-Derivate der Formel (Ia-30) gemäß Anspruch 1 auf Schädlinge und/oder ihren Lebensraum einwirken läßt.

**5.** Verwendung von Azin-Derivaten der Formel (Ia-30) gemäß Anspruch 1 zur Bekämpfung von tierischen Schädlingen.

**6.** Verfahren zur Herstellung von Schädlingsbekämpfungsmitteln, dadurch gekennzeichnet, daß man Azin-Derivate der Formel (Ia-30) gemäß Anspruch 1 mit Streckmitteln und/oder oberflächenaktiven Mitteln vermischt.

**Claims**

**1.** Azine derivatives of the formula (Ia-30)

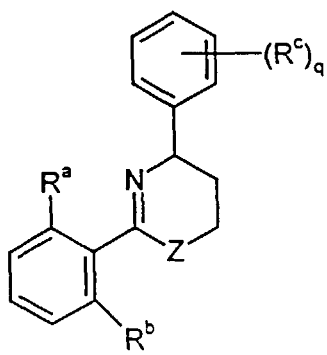

(Ia-30)

in which

$R^a$ represents hydrogen, fluorine or chlorine,
$R^b$ represents fluorine or chlorine,
q represents a number from 1 to 5,
$R^c$ represents tri($C_1$-$C_6$-alkyl)silyl, or represents $C_3$-$C_7$-cycloalkyl which is optionally mono-substituted to tris-

ubstituted by $C_1$-$C_6$-alkyl, or represents

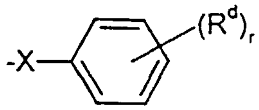

in which

X represents a direct bond, $C_1$-$C_6$-alkanediyl, $C_1$-$C_6$-oxy-alkanediyl or di($C_1$-$C_6$-alkyl)silyl where

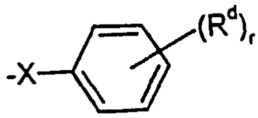

does not represent unsubstituted phenylmethoxy,

r represents a number from 0 to 5,

$R^d$ represents $C_1$-$C_{12}$-alkyl, $C_1$-$C_{12}$-alkoxy, halogen, $C_1$-$C_6$-haloalkyl, $C_1$-$C_6$-haloalkoxy or tri($C_1$-$C_6$-alkyl)silyl and

Z represents oxygen or sulphur,

with the exception of the compound of the formula

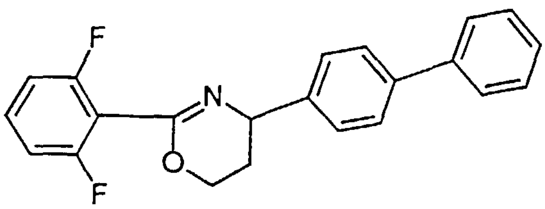

**2.** Process for the preparation of azine derivatives of the formula (Ia-30) according to Claim 1, characterized in that

a) amino alcohols of the formula

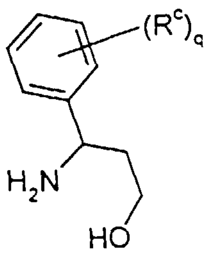

(II-30)

in which $R^c$ and q have the meanings given in Claim 1 are reacted with a carboxylic acid of the formula

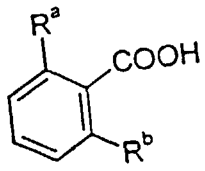

(III-30)

in which $R^a$ and $R^b$ have the meanings given in Claim 1, with a dehydrating agent, if appropriate in the presence of a diluent; or

b) amidoalcohols of the formula

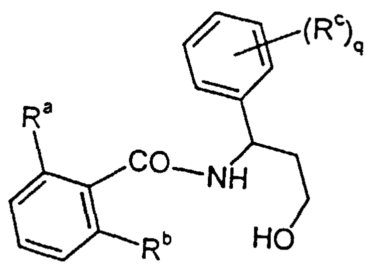

(IVa-30)

in which $R^a$, $R^b$, $R^c$ and q have the meanings given in Claim 1 are reacted with a dehydrating agent, if appropriate in the presence of a diluent; or

c) amide derivatives of the formula

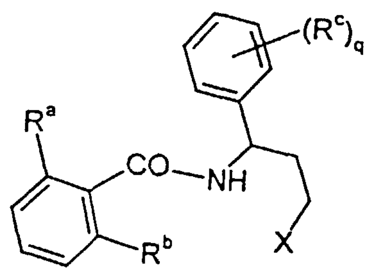

(Va-30)

in which $R^a$, $R^b$, $R^c$ and q have the meanings given in Claim 1
and
X represents halogen, alkylsulphonyloxy or arylsulphonyloxy
are reacted with a base, if appropriate in the presence of a diluent;
or

d) amidoalcohols of the formula

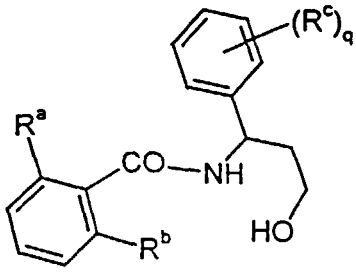

(IVa-30)

in which $R^a$, $R^b$, $R^c$ and q have the meanings given in Claim 1 are reacted with thienylating agents, if appropriate in the presence of a diluent.

3. Composition for controlling animal pests, characterized in that it comprises at least one azine derivative of the formula (Ia-30) according to Claim 1.

4. Method of controlling animal pests, characterized in that azine derivatives of the formula (Ia-30) according to Claim 1 are allowed to act on pests and/or their environment.

5. Use of azine derivatives of the formula (Ia-30) according to Claim 1 for controlling animal pests.

6. Process for the preparation of pesticides, characterized in that azine derivatives of the formula (Ia-30) according to Claim 1 are mixed with extenders and/or surfactants.

**Revendications**

1. Dérivés aziniques de formule (Ia-30)

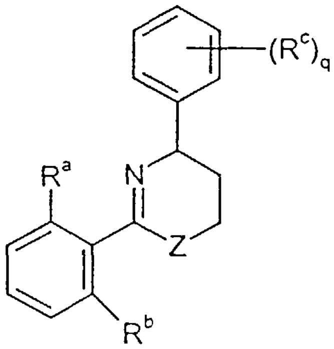

(Ia-30)

dans laquelle

$R^a$ représente l'hydrogène, le fluor ou le chlore,
$R^b$ représente le fluor ou le chlore,
q est un nombre de 1 à 5,
$R^c$ est un groupe tri(alkyle en $C_1$ à $C_6$)silyle, un groupe cycloalkyle en $C_3$ à $C_7$ éventuellement substitué une à trois fois par un reste alkyle en $C_1$ à $C_6$, ou un groupe

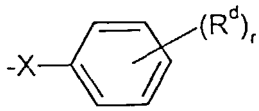

dans lequel

X est une liaison directe, un groupe alcanediyle en $C_1$ à $C_6$, oxy-alcanediyle en $C_1$ à $C_6$ ou di(alkyle en $C_1$ à $C_6$)silyle,
le groupe

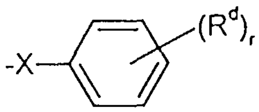

ne représentant pas un groupe phénylméthoxy non substitué,

r est un nombre de 0 à 5,

$R^d$ est un groupe alkyle en $C_1$ à $C_{12}$, alkoxy en $C_1$ à $C_{12}$, un halogène, un groupe halogénalkyle en $C_1$ à $C_6$, halogénalkoxy en $C_1$ à $C_6$ ou tri(alkyle en $C_1$ à $C_6$)silyle et

Z représente l'oxygène ou le soufre,
excepté le composé de formule

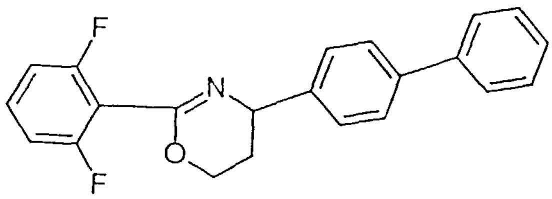

**2.** Procédé de production de dérivés aziniques de formule (Ia-30) suivant la revendication 1, caractérisé en ce que

a) on fait réagir des amino-alcools de formule

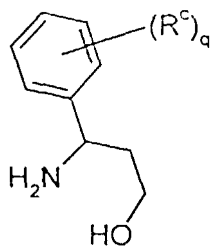

(II-30),

dans laquelle $R^c$ et q ont la définition indiquée dans la revendication 1,
avec un acide carboxylique de formule

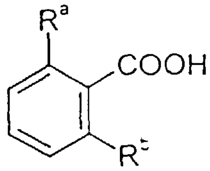

(III-30),

dans laquelle $R^a$ et $R^b$ ont les définitions indiquées dans la revendication 1, avec un agent déshydratant, le cas échéant en présence d'un diluant ;
ou bien

b) on fait réagir des amido-alcools de formule

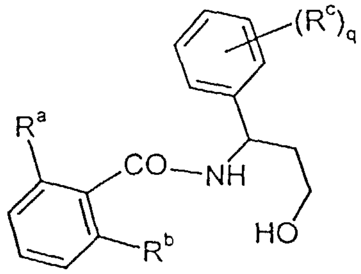

(IVa-30)

dans lesquels $R^a$, $R^b$, $R^c$ et q ont les définitions indiquées dans la revendication 1, avec un agent déshydratant, le cas échéant en présence d'un diluant ;
ou bien

c) on fait

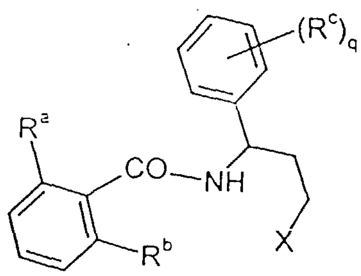

(Va-30)

réagir des dérivés d'amide de formule dans lesquels $R^a$, $R^b$, $R^c$ et q ont les définitions mentionnées dans la revendication 1,
et
X représente un halogène, un groupe alkylsulfonyloxy ou arylsulfonyloxy,
avec une base, éventuellement en présence d'un diluant ; ou bien

d) on fait réagir des amido-alcools de formule

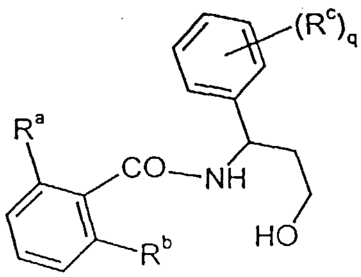

(IVa-30)

dans lesquels $R^a$, $R^b$, $R^c$ et q ont les définitions mentionnées dans la revendication 1, avec des agents de thiénylation, le cas échéant en présence d'un diluant.

**3.** Compositions destinées à combattre des parasites animaux, caractérisées par une teneur en au moins un dérivé azinique de formule (Ia-30) suivant la revendication 1.

**4.** Procédé pour combattre des parasites animaux, caractérisé en ce qu'on fait agir des dérivés aziniques de formule (Ia-30) suivant la revendication 1 sur les parasites et/ou sur leur milieu.

**5.** Utilisation de dérivés aziniques de formule (Ia-30) suivant la revendication 1 pour combattre des parasites animaux.

**6.** Procédé de préparation de compositions pesticides, caractérisé en ce qu'on mélange des dérivés aziniques de formule (Ia-30) suivant la revendication 1 avec des diluants et/ou des agents tensio-actifs.